# EUROPÄISCHE PATENTSCHRIFT

(11) **EP 2 828 600 B1**
(45) Veröffentlichungstag und Bekanntmachung des Hinweises auf die Patenterteilung: **31.03.2021**
(21) Anmeldenummer: 13706980.3
(22) Anmeldetag: 27.02.2013
(51) Int. Cl.: F28F 9/16, F28F 21/06, F28D 7/16, A61M 1/16, B01D 63/02

(54) **WÄRMETAUSCHER FÜR EINEN OXYGENATOR UND VERFAHREN ZUR HERSTELLUNG EINES DERARTIGEN WÄRMETAUSCHERS**
HEAT EXCHANGER FOR AN OXYGENATOR AND METHOD FOR PRODUCING SUCH A HEAT EXCHANGER
ÉCHANGEUR THERMIQUE POUR OXYGÉNATEUR ET SON PROCÉDÉ DE FABRICATION

(30) Priorität: 23.03.2012 DE 102012204705
(43) Veröffentlichungstag der Anmeldung: 28.01.2015
(73) Patentinhaber: Raumedic AG, 95213 Münchberg (DE)
(72) Erfinder: STÖCKER, Martin, 95233 Helmbrechts (DE); ZEITLER, Andreas, 95356 Grafengehaig (DE)
(74) Vertreter: Rau, Schneck & Hübner Patentanwälte Rechtsanwälte PartGmbB
(86) Internationale Anmeldenummer: PCT/EP2013/053853
(87) Internationale Veröffentlichungsnummer: WO 2013/139564

(56) Entgegenhaltungen:
- EP-A1- 1 714 692
- DE-A1- 1 501 595
- DE-A1- 3 636 583
- DE-C1- 3 805 414
- DE-C1- 19 652 695
- DE-U1- 9 313 138
- US-A1- 2001 016 729
- US-A1- 2001 016 729
- US-A1- 2011 270 147

## Beschreibung

Die Erfindung betrifft einen Wärmetauscher für einen Oxygenator sowie ein Verfahren zur Herstellung eines derartigen Wärmetauschers.

Ein Oxygenator ist Bestandteil eines extrakorporalen Blutkreislaufs während einer offenen Herzoperation. Eine Temperaturregelung findet in einem Wärmetauscher des Oxygenators statt, indem ein Wärmeaustausch zwischen Blut und einem Wärmetauschermedium erfolgt.

Aus der DE 10 2010 000 820 A1 ist ein Wärmetauscher bekannt, der mehrere Schlauchabschnitte aufweist, die durch quer verlaufende Wirknähte, sogenannte Stützfäden, zu einer Matte miteinander verwirkt sind. Die Matte wird zu einem Wärmetauscherkörper gerollt und daraus ein Wärmetauscher hergestellt. Weitere Wärmetauscher mit verwirkten Schlauchmatten sind aus der DE 28 25 065 A1 und aus der WO 2011/139392 A1 bekannt. Die DE 93 13 138 U1, die US 2011/0270147 A1 und die DE 15 01 595 A1 offenbaren jeweils eine Schlauchmatte, bei der einzelne Schlauchabschnitte mit einem Trägermaterial verbunden und so zu einer Schlauchmatte gefügt sind. Die Schlauchmatte wird zu einem Schlauchmattenbündel gerollt, in ein Gehäuse eingeführt und dort vergossen.

Die DE 36 36 583 A1, die DE 38 05 414 C1, die US 2001/0016729 A1 und die DE 196 52 695 C1 offenbaren jeweils einen Wärmetauscher, bei dem Schlauchabschnitte als loses Bündel in ein Gehäuse eingeführt werden. Um einen definierten Abstand zwischen den Schlauchabschnitten zu gewährleisten, sind zusätzliche Abstandsmittel erforderlich.

Die EP 1 714 692 A1 offenbart einen Dialysefilter, bei dem Schlauchabschnitte in einem Gehäuse angeordnet sind. Die Schlauchabschnitte sind an ihren Enden in einer Vergussmasse eingebettet. Die Vergussmasse ist mit dem Gehäuse nicht verbunden. Zum Abdichten des Bündels in dem Gehäuse sind zusätzliche Dichtungsringe erforderlich.

Es ist nachteilig, dass ein Wärmetauscher, insbesondere dafür verwendete Schlauchabschnitte, während der Herstellung verschmutzt und/oder beschädigt werden können.

Es ist eine Aufgabe der vorliegenden Erfindung, einen Wärmetauscher zu verbessern, insbesondere derart, dass der Wärmetauscher unkompliziert herstellbar ist und somit insbesondere das Risiko einer Verschmutzung und/oder Beschädigung während der Herstellung reduziert ist.

Diese Aufgabe ist erfindungsgemäß gelöst durch einen Wärmetauscher mit den im Anspruch 1 angegebenen Merkmalen.

Erfindungsgemäß wurde erkannt, dass das Risiko von Verschmutzung und/oder Beschädigung während der Herstellung eines Wärmetauschers reduziert ist, wenn Schlauchabschnitte miteinander durch Fügen mittels chemischer und/oder physikalischer Klebstoffverbindungen verbunden sind. Dadurch ist es insbesondere nicht erforderlich, die Schlauchabschnitte in einem Wirkverfahren miteinander zu verbinden. Insbesondere kann dadurch ausgeschlossen werden, dass die Schlauchabschnitte durch die Wirknähte abgeschnürt werden und somit eine Strömungsfläche im Schlauchinneren, also die Schlauchquerschnittsfläche, reduziert wird. Je nach Funktionsprinzip kann der Wärmetauscher derart genutzt werden, dass Blut im Inneren der Schlauchabschnitte und Wärmetauschermedium in zwischen den Schlauchabschnitten gebildeten Zwischenräumen fließt oder umgekehrt, also Wärmetauschermedium im Inneren der Schlauchabschnitte und Blut in den Zwischenräumen. Die einzelnen Strömungsquerschnitte der Schlauchabschnitte werden durch das erfindungsgemäße Fügen mittels chemischer und/oder physikalischer Klebstoffverbindungen nicht beeinträchtigt. Gleichzeitig sind die einzelnen Schlauchabschnitte zu einem Bündel sicher fixiert und miteinander verbunden. Das bedeutet, dass durch das erfindungsgemäße Kleben der einzelnen Schlauchabschnitte miteinander ein eigenständiges, in sich formstabiles Bündel gebildet wird. Das Bündel ist im Wesentlichen zylinderförmig. Die Handhabung und insbesondere ein nachfolgender Fügevorgang des Bündels in einem Gehäuse eines Wärmetauschers ist vereinfacht. Das Bündel insgesamt, und nicht die einzelnen Schlauchabschnitte, kann mit dem Gehäuse verbunden und insbesondere verklebt werden. Ein derartiges Herstellungsverfahren ist besonders robust und fehlerunanfällig. Weiterhin weist der Wärmetauscher eine reduzierte Verschmutzung auf. Insbesondere kann ausgeschlossen werden, dass ein Abrieb eines Stützfadens, der in Folge eines Wirkens der Schlauchabschnitte zu einer Matte auftreten würde, zu einer Verstaubung des Wärmetauschers führt. Der Wärmetauscher kann umkomplizierter und insbesondere zuverlässiger hergestellt werden. Die Herstellung des Wärmetauschers ist besonders wirtschaftlich, da die Ausschussrate, also die Anzahl der nicht zu gebrauchenden hergestellten Wärmetauscher reduziert ist. Weiterhin ermöglicht der Wärmetauscher eine verbesserte Anwendung, da eine gleichmäßigere Strömung des Blutes zwischen den Schlauchabschnitten gewährleistet ist. Insbesondere ist eine Strömung des Blutes zwischen den Schlauchabschnitten nicht durch Stützfäden gestört. Dadurch können Verwirbelungen des Blutes vermieden werden. Da entlang des Strömungsweges des Blutes in dem Wärmetauscher keine Strömungshindernisse vorliegen, kann das Blut mit im Wesentlichen konstantem Druck durch den Wärmetauscher gepumpt werden. Insbesondere ist es nicht erforderlich, einen erhöhten Pumpdruck zur Überwindung von Engstellen und/oder Hindernissen in dem Wärmetauscher zu überwinden. Dadurch wird sichergestellt, dass bei der Anwendung des Wärmetauschers eine verringerte Blutschädigung auftritt. Der Wärmetauscher umfasst mehrere jeweils eine Schlauch-Längsachse aufweisende Schlauchabschnitte. Die Schlauchabschnitte sind in dem eine Bündel-Längsachse aufweisenden Bündel angeordnet. Das Bündel weist mindestens einen Verbindungsabschnitt auf, in dem die Schlauchabschnitte durch Fügen miteinander verbunden sind. Die Schlauchabschnitte sind durch Kleben miteinander verbunden. Die Verbindung durch Kleben ist schnell und unkompliziert herstellbar und ermöglicht hohe und dauerhaft zuverlässige Klebeverbindungen der Schlauchabschnitte miteinander. Die Schlauchabschnitte sind ausschließlich durch Kleben miteinander verbunden. Der Aufbau des Wärmetauschers ist robust. Insbesondere ist eine Weiterverarbeitung eines geklebten Bündels und die Handhabung vereinfacht. Das Bündel weist eine senkrecht zur Bündel-Längsachse orientierte, insbesondere kreisförmige Querschnittsfläche auf. Es ist auch möglich, die Schlauchabschnitte je nach Anwendung in einer davon verschiedenen Bündelform, d. h. mit einer von einer Kreisform verschiedenen Querschnittsfläche senkrecht zur Bündel-Längsachse, anzuordnen. Dadurch, dass bei der Fixierung der Schlauchabschnitte zu einem Bündel auf Wirkfäden verzichtet werden kann, können die einzelnen Schlauchabschnitte der Strömungsbewegung beispielsweise frei schwingend oder pendelnd folgen. Dadurch wird die Strömung des Bluts bzw. des Wärmetauschermediums nicht beeinträchtigt. Die Strömungsqualität ist zusätzlich verbessert und führt zu einer verbesserten Durchmischung der Medien. Dadurch ist der Wirkungsgrad des Wärmetauschers erhöht.

Der erfindungsgemäße Wärmetauscher weist eine erhöhte Stabilität und Steifigkeit auf. Dadurch, dass der mindestens eine Verbindungsabschnitt Klebstoff aufweist, ist er zusätzlich stabilisiert und insbesondere starr. Als Klebstoff kann insbesondere ein Gießharz und insbesondere Acrylat, Phenolharz, Silikon, Polyurethan oder Copolymer verwendet werden. Es ist auch möglich, als Klebstoff eine thermoplastische Gießmasse zu verwenden. Ein Acrylat ist beispielsweise mittels UV-Strahlung aushärtenbar. Es ist aber auch möglich, eine Vernetzung durch additionsvernetzende oder radikalisch vernetzende Gießharze zu erreichen. Es ist auch eine Polykondensationsreaktion zur Vernetzung oder eine katalytische Vernetzung wie beispielsweise platinkatalysiertes Silikon möglich. Bei thermoplastischen Gießmassen wie beispielsweise Polyurethan erfolgt keine Vernetzung. Ein Wärmetauscher, bei dem das Bündel zumindest abschnittsweise in einem Gehäuse angeordnet ist, ist robust. Das Bündel ist innerhalb des Gehäuses geschützt. Das Gehäuse ist insbesondere aus Kunststoff und insbesondere aus transparentem Kunststoff hergestellt.

Ein Wärmetauscher gemäß der Erfindung mit zwei Verbindungsabschnitten, die jeweils an einem Bündelende angeordnet sind, weist eine erhöhte Stabilität auf. Dadurch, dass jeweils an einem Bündelende ein Verbindungsabschnitt vorgesehen ist, an welchem die Schlauchabschnitte miteinander verbunden sind, weist das Bündel eine Eigenstabilität auf. Insbesondere ist es dadurch möglich, eine Anordnung der Schlauchabschnitte zu dem Bündel mit einer gewünschten Orientierung der Schlauch-Längsachsen zu konservieren.

Der Wärmetauscher gemäß der Erfindung, bei dem das Bündel an den Verbindungsabschnitten über ihre äußere Zylindermantelfläche an einer Innenseite des Gehäuses verklebt ist, ist besonders robust und stabil. Dadurch, dass das Bündel mit dem Gehäuse verbunden ist, ist eine zuverlässige und insbesondere störungsfreie Anwendung des Wärmetauschers in dem Oxygenator gewährleistet. Dadurch, dass das Bündel, insbesondere an dem Verbindungsabschnitt, mit dem Gehäuse verbunden ist, ist ausgeschlossen, dass die Schlauchabschnitte während des Verbindens des Bündels mit dem Gehäuse beeinträchtigt, insbesondere verschmutzt und/oder beschädigt werden.

Ein Wärmetauscher nach Anspruch 2 weist eine verbesserte Blutkompatibilität auf. Darüber hinaus weisen die Schlauchabschnitte aus Kunststoff, die insbesondere aus Polyurethan hergestellt sein können, eine geringe Masse auf. Ein einzelner Schlauchabschnitt wiegt typischerweise etwa 0,035 g. Die zu verbindenden Schlauchabschnitte, also das Bündel ohne die Verbindungsabschnitte, wiegen höchstens 200 g, wobei die Masse je nach Größe des Bündels, d.h. in Abhängigkeit der Anzahl und/oder der Länge der Schlauchabschnitte abweichen kann. Die Schlauchabschnitte weisen eine hohe mechanische Flexibilität auf und ermöglichen eine erhöhte Gestaltungsvielfalt bei der Herstellung des Wärmetauschers und insbesondere bei der Gestaltung des Bündels.

Ein Wärmetauscher gemäß Anspruch 3 weist einen verbesserten Wirkungsgrad auf. Dadurch, dass die Schlauchabschnitte derart angeordnet sind, dass deren Schlauch-Längsachsen mit der Bündel-Längsachse einen Winkel einschließen, der größer ist als 10°, insbesondere größer als 20° und insbesondere größer als 30°, ist die Strömung des Blutes oder des Wärmetauschermediums, insbesondere Wasser, an den Außenseiten der Schlauchabschnitte verbessert. Die Schlauchabschnitte, durch die das jeweils andere Medium strömt, werden durch das Blut bzw. das Wärmetauschermedium besser umströmt, so dass die Kontaktfläche, d. h. die Wärmetauscherfläche, erhöht ist. Der Wärmeaustausch zwischen dem Blut und dem Wärmetauschermedium ist verbessert. Insbesondere sind die Schlauchabschnitte derart angeordnet, dass die Schlauch-Längsachsen zueinander parallel orientiert sind.

Bei einem Wärmetauscher gemäß Anspruch 4 ist der Wirkungsgrad des Wärmetauschers zusätzlich erhöht. Dadurch, dass die Schlauchabschnitte mit den Schlauch-Längsachsen zumindest abschnittsweise nicht-linear und insbesondere gekrümmt angeordnet sind, ist das Umströmen der Schlauchabschnitte verbessert. Die Schlauchabschnitte weisen insbesondere einen Biegeradius auf. Insbesondere ist es denkbar, dass die Schlauchabschnitte entlang des Bündels wellenförmig oder sinuskurvenartig ausgeführt sind mit einem Wellenberg und einem Wellental.

Ein Wärmetauscher nach Anspruch 5 ermöglicht verbesserte Strömungseigenschaften für das Blut bzw. das Wärmetauschermedium. Dadurch ist der Wirkungsgrad des Wärmetauschers verbessert. Das Bündel hat eine runde Querschnittsfläche. Es ist auch denkbar, dass das Bündel beispielsweise eine ovale, rechteckige, quadratische oder eine andere vieleckförmige Querschnittsfläche aufweist.

Ein Wärmetauscher nach Anspruch 6 weist eine erhöhte Schlauchabschnitts-Dichte auf, d. h. die Anzahl der Schlauchabschnitte in dem Bündel ist erhöht. Dadurch, dass Wirkfäden nicht erforderlich sind, können die einzelnen Schlauchabschnitte dichter angeordnet werden, d. h. ein senkrechter Abstand zwischen Außenwänden zweier benachbarter Schlauchabschnitte kann reduziert werden. Der senkrechte Abstand beträgt höchstens 200 µm und insbesondere höchstens 100 µm. Die Schlauchwanddicke beträgt etwa 0,01 mm bis 0,2 mm. Ein Innendurchmesser des Schlauchabschnitts beträgt etwa 0,1 mm bis 2,0 mm. Entsprechend beträgt ein Außendurchmesser des Schlauchabschnitts, der sich aus der Summe der Innendurchmessers und der doppelten Wandstärke ergibt, etwa 0,12 mm bis 2,4 mm. Dadurch, dass vergleichsweise mehr Schlauchabschnitte in einem Bündel angeordnet werden können, kann die Wärmetauscheroberfläche bei gleicher Bündelgröße, d. h. bei gleicher Querschnittsfläche des Bündels, erhöht werden. Alternativ kann bei vorgegebener Wärmetauscherleistung die Länge des Wärmetauschers entlang der Bündel-Längsachse reduziert werden, wobei die Gesamtwärmetauscheroberfläche konstant bleibt. Aufgrund der reduzierten Wärmetauscherlänge kann der Durchflusswiderstand durch den Wärmetauscher reduziert werden, wodurch eine Verbesserung der Blutkompatibilität erreicht wird.

Es ist eine weitere Aufgabe der vorliegenden Erfindung, ein Verfahren zur Herstellung eines Wärmetauschers für einen Oxygenator zu verbessern, so dass insbesondere dessen Herstellung zuverlässiger und unkomplizierter möglich ist und der derart hergestellte Wärmetauscher insbesondere verbesserte Anwendungseigenschaften aufweist.

Diese Aufgabe ist erfindungsgemäß gelöst durch ein Verfahren mit den in Anspruch 7 angegebenen Merkmalen.

Erfindungsgemäß wurde erkannt, dass Schlauchabschnitte, die in einem Bündel anzuordnen sind, miteinander durch ein Fügeverfahren mittels chemischer und/oder physikalischer Klebstoffverbindungen miteinander zu verbinden sind. Dadurch ist es möglich, dass ein mechanisches Verbinden wie beispielsweise ein Verwirken der Schlauchabschnitte zu einer Matte und ein anschließendes Rollen der Matte auf einen Kern zu einem Bündel, nicht erforderlich ist. Dadurch ist es insbesondere möglich, die zusätzlichen Verfahrensschritte wie Wirken der Schlauchabschnitte zu einer Matte und Wickeln der Matte zu einem Bündel, zu vermeiden. Das Verfahren ist unkompliziert und schnell durchführbar. Darüber hinaus kann das Risiko einer möglichen Beschädigung der Schlauchabschnitte durch Abschnüren durch die Wirkfäden und/oder durch eine Vordehnung, die insbesondere bis zu einem Reißen, der Schlauchabschnitte führen kann, vermieden werden. Eine Verschmutzung der Schlauchabschnitte, insbesondere eine Verstaubung während des Verwirkens der Schlauchabschnitte durch Abrieb des Wirkfadens, ist ebenfalls ausgeschlossen. Ein durch dieses Verfahren hergestellter Wärmetauscher weist weitere Vorteile auf, die vorstehend bereits anhand des Wärmetauschers erläutert worden sind und auf die hiermit verwiesen wird. Bei dem Verfahren werden mehrere Schlauchabschnitte bereitgestellt, die beispielsweise in einer bestimmten Länge zugeschnitten zur Verfügung gestellt sind. Die Schlauchabschnitte werden in einem Bündel angeordnet, wobei die Schlauch-Längsachsen der Schlauchabschnitte insbesondere parallel zueinander orientiert sind. Dabei ist es denkbar, dass die Schlauchabschnitte in einer hülsenförmigen Halterung zu dem Bündel angeordnet sind. Es ist auch möglich, dass die Schlauchabschnitte von einem Greifwerkzeug, insbesondere einer Greifzange, aktiv gehalten werden, wobei die Greifzange eine dem Bündel entsprechende Form aufweist.

Bei einem Verfahren bei dem das Einführen des Bündels in ein Gehäuse derart erfolgt, dass jeweils ein an einem Bündelende angeordneter Verbindungsabschnitt zumindest innerhalb des Gehäuses angeordnet ist, wird ein vorgefertigtes Bündel in ein, insbesondere rohrförmiges, Gehäuse eingeführt. Ein vorgefertigtes Bündel weist beispielsweise an den Bündelenden miteinander verbundene, insbesondere verklebte, Schlauchabschnitte auf, die nach dem Verbinden um die Bündel-Längsachse tordiert und/oder entlang der Bündel-Längsachse gestaucht worden sind. Insbesondere ist eine Außenform des Bündels, beispielsweise eine Zylinderform, an eine Innenkontur des Gehäuses angepasst.

Ein Verfahren nach Anspruch 8 ermöglicht eine schnelle und unkomplizierte Anordnung der Schlauchabschnitte in einer gewünschten Anordnung. Dadurch ist es möglich, die einzelnen Schlauchabschnitte bezüglich einer Bündel-Längsachse in einem von 0° verschiedenen Winkel anzuordnen, der insbesondere größer ist als 10°, insbesondere größer als 20° und insbesondere größer als 30°. Alternativ oder zusätzlich können die Schlauchabschnitte entlang der Bündel-Längsachse gekrümmt angeordnet werden. Insbesondere ist es vorteilhaft, wenn die beiden, einander gegenüberliegenden Bündelenden zunächst, insbesondere durch Kleben, miteinander verbunden werden und anschließend ein Tordieren des einen Bündelendes um die Bündel-Längsachse und/oder ein Stauchen des Bündels entlang der Bündel-Längsachse erfolgt.

Bei einem Verfahren nach Anspruch 9 erfolgt das Einführen des Bündels in das Gehäuse derart, dass jeweils ein an einem Bündelende angeordneter Verbindungsabschnitt zumindest abschnittsweise außerhalb des Gehäuses angeordnet ist. Dadurch ist die Handhabung des Bündels während des Einführens in das Gehäuse vereinfacht. Insbesondere ist es möglich, die an dem Gehäuse überstehenden Bereiche der Verbindungsabschnitte abzutrennen und damit einen, insbesondere bündigen, Abschluss des Bündels an dem Gehäuse zu schaffen.

Ein Verfahren nach Anspruch 10 vereinfacht die Herstellung mehrerer Bündel. Ein derartiges Verfahren ist insbesondere für die Massenproduktion besonders geeignet, da mehrere Bündel gleichzeitig herstellbar sind. Es ist beispielsweise denkbar, dass Schlauchabschnitte mit großen Längen, die insbesondere einem Vielfachen einer gewünschten Länge eines Wärmetauschers entsprechen, in Bündelform angeordnet sind, wobei die Schlauchabschnitte in einer Wärmetauscherlänge miteinander verbunden sind. Insbesondere ist es also denkbar, dass Schlauchabschnitte in regelmäßigen Abständen in einem Verbindungsabschnitt miteinander verbunden sind und nachfolgend ein Auftrennen eines derartigen "Endlos"-Bündels an den Verbindungsabschnitten erfolgt, so dass mehrere Bündel mit jeweils einem an den Bündelenden angeordneten Verbindungsabschnitt zur Verfügung steht.

Ausführungsbeispiele der Erfindung werden nachfolgend anhand der Zeichnung näher erläutert. In dieser zeigen:
- Fig. 1: eine Seitenansicht eines erfindungsgemäßen Wärmetauschers gemäß einer ersten Ausführungsform,
- Fig. 2: eine Schnittdarstellung gemäß Linie II-II in Fig. 1,
- Fig. 3: eine Fig. 1 entsprechende Ansicht eines Bündels in einem Gehäuse,
- Fig. 4: eine Fig. 1 entsprechende Ansicht eines Wärmetauschers gemäß einer weiteren Ausführungsform, und
- Fig. 5: eine Fig. 2 entsprechende vergrößerte Detaildarstellung von Schlauchabschnitten des Bündels.

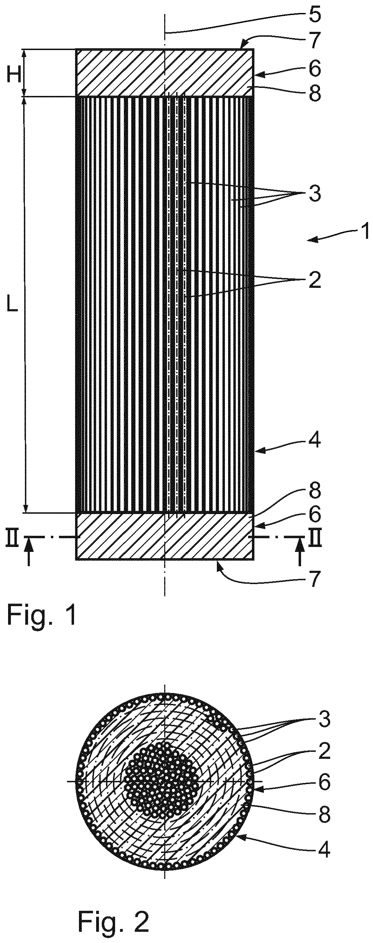

Ein in den Fig. 1 bis 3 dargestellter Wärmetauscher 1 umfasst mehrere jeweils eine Schlauch-Längsachse 2 aufweisende Schlauchabschnitte 3. Die Schlauchabschnitte 3 sind aus Kunststoff und insbesondere aus Polyurethan (PUR) hergestellt. Es sind auch andere Kunststoffe zur Herstellung der Schlauchabschnitte 3 möglich wie beispielsweise Polyester, insbesondere Polyethylenterephthalat (PET) oder Polybutylenterephthalat (PBT), Polyamide und Copolymere oder Polyurethane und Copolymere, insbesondere Polyether-Polyurethan oder Polyester-Polyurethan.

Gemäß dem gezeigten Ausführungsbeispiel sind die Schlauchabschnitte 3 zu einem Bündel 4 angeordnet, das eine Bündel-Längsachse 5 aufweist. Die Schlauchabschnitte 3 sind derart angeordnet, dass deren Schlauch-Längsachsen 2 jeweils parallel zueinander und parallel zur Bündel-Längsachse 5 orientiert sind.

Die Schlauchabschnitte 3 sind in zwei Verbindungsabschnitten 6, die jeweils an Bündelenden 7 angeordnet sind, durch Kleben, also durch Fügen mittels chemischer und/oder physikalischer Klebstoffverbindungen, miteinander verbunden. Gemäß der gezeigten Ausführungsform sind die Schlauchabschnitte 3 ausschließlich durch einen Klebstoff 8 in dem jeweiligen Verbindungsabschnitt 6 verbunden. Das bedeutet, dass die einzelnen Schlauchabschnitte 3 direkt miteinander verbunden sind.

Das Bündel 4 weist eine im Wesentlichen zylindrische Grundform auf. Eine senkrecht zur Bündel-Längsachse 5 orientierte Querschnittsfläche ist kreisförmig. Grundsätzlich ist es auch denkbar, dass andere Grundformen für das Bündel 4 verwendet werden. Die Verbindungsabschnitte 6 sind ebenfalls zylinderförmig ausgeführt und weisen eine entlang der Bündel-Längsachse 5 orientierte Höhe H auf. Gemäß dem gezeigten Ausführungsbeispiel beträgt die Höhe H 5 mm.

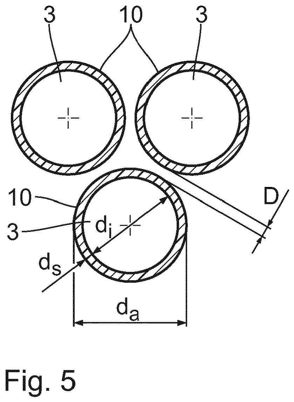

Der Wärmetauscher 1 weist eine Länge L auf, die durch die beiden gegenüberliegend an den Bündelenden 7 angeordneten Verbindungsabschnitte 6 begrenzt ist. Innerhalb der Länge L erfolgt der Wärmeaustausch zwischen dem Wärmetauschermedium, insbesondere Wasser, und dem zwischen den Schlauchabschnitten, insbesondere entgegen der Strömungsrichtung des Wärmetauschermediums gerichtet strömenden Bluts. Gemäß dem gezeigten Ausführungsbeispiel beträgt die Länge L etwa 170 mm. Die Länge L des Wärmetauschers 1, die Anzahl der zu dem Bündel 4 angeordneten Schlauchabschnitte 3, der jeweilige Schlauchinnendurchmesser der Schlauchabschnitte 3 und die jeweilige Wanddicke der Schlauchabschnitte 3 wird entsprechend den Anforderungen an den Wärmetauscher 1 gewählt. Gemäß der Detaildarstellung des Ausführungsbeispiels in Fig. 5 beträgt ein Innendurchmesser dᵢ = 0,67 mm, ein Außendurchmesser dₐ = 0,79 mm und eine Schlauchwanddicke dₛ = 0,06 mm. Insbesondere die Anzahl der verwendeten Schlauchabschnitte 3 kann abhängig von der Patientengröße, d. h. des Volumens des umzuwälzenden Blutes, variieren.

Das Bündel 4 weist entlang der Bündel-Längsachse 5 eine konstante Querschnittsfläche auf. Insbesondere sind die jeweiligen Querschnittsflächen an den beiden Bündelenden 7 gleich groß. Ein senkrechter Abstand D zwischen Außenwänden 10 zwei benachbarter Schlauchabschnitte 3 beträgt höchstens 200 µm. Insbesondere Beträgt der Abstand D höchstens 100 µm. Dadurch ist gewährleistet, dass die Schlauchabschnitte 3 mit einer vergrößerten Dichte zu dem Bündel 4 zusammengefasst werden.

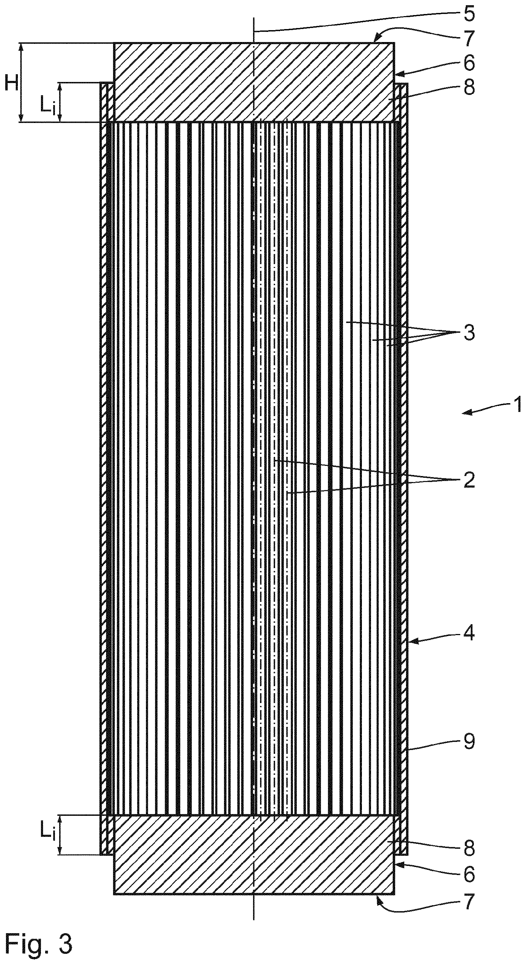

Wie in Fig. 3 dargestellt, wird das Bündel 4 zumindest abschnittsweise in einem Gehäuse 9 angeordnet. Dabei ragen die Verbindungsabschnitte 6 jeweils zur Hälfte, also mit etwa 50% der Höhe H, an dem Gehäuse 9 hervor. Das bedeutet, dass ein innerer Abschnitt mit einer Länge Lᵢ des Verbindungsabschnitts 6 innerhalb des Gehäuses 9 angeordnet ist. An dem, zylindrisch ausgeführten, inneren Bereich der Verbindungsabschnitte 6 sind die über ihre äußere Zylindermantelfläche an einer Innenseite des Gehäuses 9 verklebt. Insbesondere dient dazu der gleiche Klebstoff 8 wie zur Verbindung der einzelnen Schlauchabschnitte 3. Die Länge Lᵢ beträgt etwa 50% der Höhe H des Verbindungsabschnitts 6, also etwa 2,5 mm und gewährleistet damit ein sicheres Verbinden des Bündels 4 in dem Gehäuse 9.

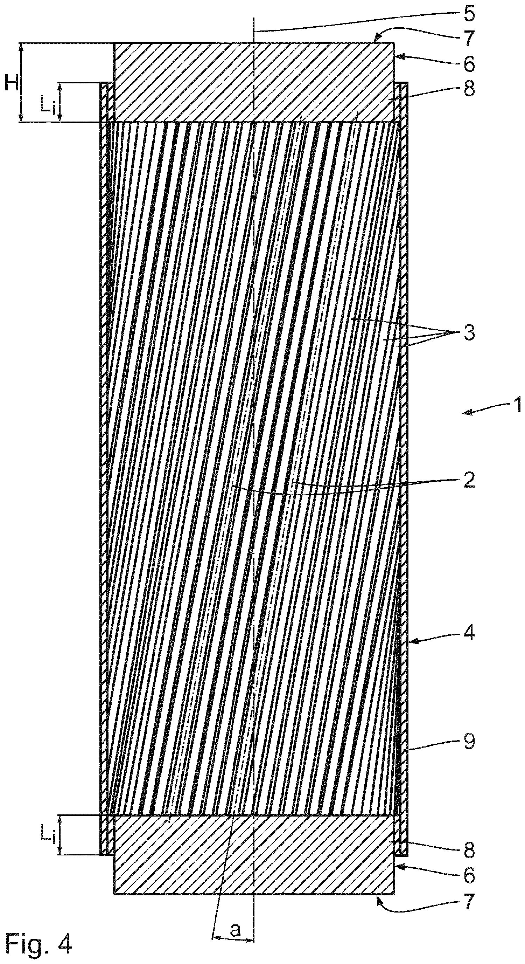

Nachfolgend wird anhand der Darstellung in Fig. 4 eine weitere Ausführungsform eines Wärmetauschers beschrieben. Identische Teile, die anhand der Fig. 1 bis 3 bereits erläutert worden sind, erhalten identische Bezugszeichen und werden nicht nochmals im Einzelnen erläutert.

Der wesentliche Unterschied des Wärmetauschers 1 gemäß der weiteren Ausführungsform besteht darin, dass die Schlauchabschnitte 3 derart angeordnet sind, dass die Schlauch-Längsachsen 2 mit der Bündel-Längsachse 5 einen Winkel a einschließen, der insbesondere größer ist als 10°, insbesondere größer als 20° und insbesondere größer als 30°. Dadurch ist gewährleistet, dass das Strömungsverhalten des Bluts durch den Wärmetauscher 1 verbessert ist. Zusätzlich oder alternativ ist es möglich, dass die Schlauchabschnitte 3 derart in dem Bündel 4 angeordnet sind, dass die Schlauch-Längsachsen 2 zumindest abschnittsweise nicht-linear und insbesondere gekrümmt bezogen auf die Bündel-Längsachse 5 angeordnet sind.

Nachfolgend wird ein Verfahren zur Herstellung eines erfindungsgemäßen Wärmetauschers 1 näher erläutert. Zunächst werden mehrere Schlauchabschnitte 3 mit vorzugsweise identischen Längen bereitgestellt. Je nach erforderlicher Wärmetauscherkapazität kann dabei die Anzahl und Länge der Schlauchabschnitte 3 variieren. Die Schlauchabschnitte 3 werden in einer hülsenförmigen Halterung, insbesondere einem Zylinderrohr, zu einem Bündel 4 angeordnet, wobei die Schlauchabschnitte 3 vorzugsweise mit den Schlauch-Längsachsen 2 parallel zur Bündel-Längsachse 5 orientiert sind. Anschließend erfolgt ein Verbinden der Schlauchabschnitte 3 in dem mindestens einen Verbindungsabschnitt 6, insbesondere in zwei Verbindungsabschnitten 6, die jeweils an einem Bündelende 7 des Bündels 4 angeordnet sind. Das Verbinden der Schlauchabschnitte 3 miteinander erfolgt durch Kleben, insbesondere mittels Acrylharz. Dadurch, dass die Schlauchabschnitte 3 miteinander verklebt sind, werden die Schlauchenden hermetisch verschlossen und sind somit gegen eine innere Verschmutzung geschützt. Anschließend wird das verklebte Bündel 4 in das Gehäuse 9 eingeführt, wobei die beiden Verbindungsabschnitte 6 jeweils stirnseitig an dem rohrförmigen Gehäuse 9 hervorstehen. Um eine Stauchung und/oder Krümmung der Schlauchabschnitte 3 des Bündels 4 zu gewährleisten, wird zunächst ein erster Verbindungsabschnitt 6 mit dem im Gehäuse 9 verbliebenen inneren Abschnitt des Verbindungsabschnitts 6 an einer äußeren Zylindermantelfläche in dem Gehäuse 9 verklebt. Dadurch erfolgt eine Abdichtung und Versiegelung des Bündels 4 in dem Gehäuse 9. Die Klebefläche ist groß und gewährleistet eine sichere Befestigung des ersten Verbindungsabschnitts 6 in dem Gehäuse. Das Bündel 4 kann nun um die Bündel-Längsachse 5 tordiert werden, so dass die Schlauchabschnitte 3 mit den Schlauch-Längsachsen 3 in dem Winkel a zur Bündel-Längsachse 5 angeordnet sind. Zusätzlich oder alternativ ist es möglich, das Bündel 4 entlang der Bündel-Längsachse 5 zu stauchen, so dass die Schlauchabschnitte 3 zumindest abschnittsweise eine Krümmung aufweisen. Dazu wird der zweite, freie Verbindungsabschnitt 6 gegenüber dem im Gehäuse 9 fixierten, ersten Verbindungsabschnitt gestaucht und/oder tordiert werden. Der freie Verbindungsabschnitt 6 wird an dem an dem Gehäuse hervorstehenden äußeren Abschnitt mittels eines Greifwerkzeugs gehalten. Sobald der zweite Verbindungsabschnitt 6 in eine gewünschte Position verlagert worden ist, kann er entlang ebenfalls einer äußeren Zylindermantelfläche an einer Innenseite des Gehäuses 9 verklebt werden. Alternativ ist es auch möglich, eine Positionierung der Verbindungsabschnitte 6 zueinander dadurch zu erreichen, dass beide Verbindungsabschnitte 6 frei beweglich gegenüber dem Gehäuse 9 mittels je eines Greifwerkzeugs relativ zueinander positioniert und anschließend in dem Gehäuse 9 verklebt werden. Nach dem Verkleben des Bündels 4 mit dem Gehäuse 9, werden die über das Gehäuse 9 überstehenden Teile der Verbindungsabschnitte 6 abgetrennt. Dadurch werden die Enden der Schlauchabschnitte 3 wieder geöffnet.

## Patentansprüche

1. Wärmetauscher für einen Oxygenator, umfassend mehrere jeweils eine Schlauch-Längsachse (2) aufweisende Schlauchabschnitte (3), wobei
- die Schlauchabschnitte (3) in einem eine Bündel-Längsachse (5) aufweisenden Bündel (4) angeordnet sind und zwischen den Schlauchabschnitten (3) Zwischenräume gebildet sind,
- die Schlauchabschnitte (3) in mindestens einem Verbindungsabschnitt (6) des Bündels (4) durch Fügen mittels chemischer und/oder physikalischer Klebstoffverbindungen miteinander verbunden sind
- zwei Verbindungsabschnitte (6) vorgesehen sind, die jeweils an einem Bündelende (7) angeordnet sind,
wobei die Verbindungsabschnitte (6) zylindrisch ausgeführt sind,
- das Bündel (4) zumindest abschnittsweise in einem Gehäuse (9) angeordnet ist,
- die einzelnen Schlauchabschnitte (3) durch Kleben direkt miteinander verbunden sind, wodurch das Bündel (4) eigenständig und in sich formstabil ausgebildet wird,
- die Zwischenräume frei von Strömungshindernissen sind,
**dadurch gekennzeichnet, dass**
- das vorgefertigte, formstabile Bündel (4) an den Verbindungsabschnitten (6) über ihre äußeren Zylindermantelflächen an einer Innenseite des Gehäuses (9) verklebt ist.

2. Wärmetauscher nach Anspruch 1, **dadurch gekennzeichnet, dass** die Schlauchabschnitte (3) aus Kunststoff, insbesondere aus Polyurethan, hergestellt sind.

3. Wärmetauscher nach einem der vorstehenden Ansprüche, **dadurch gekennzeichnet, dass** die Schlauch-Längsachsen (2) mit der Bündel-Längsachse (5) einen Winkel (a) einschließen, der größer ist als 10°, insbesondere größer als 20° und insbesondere größer als 30°.

4. Wärmetauscher nach einem der vorstehenden Ansprüche, **dadurch gekennzeichnet, dass** die Schlauch-Längsachsen (2) zumindest abschnittsweise nicht-linear, insbesondere gekrümmt, angeordnet sind.

5. Wärmetauscher nach einem der vorstehenden Ansprüche, **dadurch gekennzeichnet, dass** das Bündel (4) zylindrisch ausgeführt ist und insbesondere entlang der Bündel-Längsachse (5) eine unveränderliche Querschnittsfläche aufweist.

6. Wärmetauscher nach einem der vorstehenden Ansprüche, **gekennzeichnet durch** einen senkrechten Abstand (D) von Außenwänden (10) zwei benachbarter Schlauchabschnitte (3), der höchstens 200 µm, insbesondere höchstens 100 µm beträgt.

7. Verfahren zur Herstellung eines Wärmetauschers gemäß einem der vorstehenden Ansprüche, umfassend die Verfahrensschritte
- Bereitstellen mehrerer jeweils eine Schlauch-Längsachse (2) aufweisende Schlauchabschnitte (3),
- Anordnen der Schlauchabschnitte (3) zu einem eine Bündel-Längsachse (5) aufweisenden Bündel (4) mit zwischen den Schlauchabschnitten (3) gebildeten Zwischenräumen, die frei von Strömungshindernissen sind,
- Verbinden der Schlauchabschnitte (3) miteinander an mindestens einem Verbindungsabschnitt (6) des Bündels (4) durch Fügen der Schlauchabschnitte (3) mittels chemischer und/oder physikalischer Klebstoffverbindungen
- Vorsehen eines zweiten solchen Verbindungsabschnittes,
**dadurch gekennzeichnet, dass**
- die einzelnen Schlauchabschnitte (3) durch Kleben direkt miteinander verbunden werden, so dass das Bündel (4) eigenständig und in sich formstabil ausgebildet wird,
- ein Einführen des vorgefertigten, formstabilen Bündels (4) in ein Gehäuse (9) derart erfolgt, dass jeweils ein an einem Bündelende (7) angeordneter Verbindungsabschnitt (6) zumindest abschnittsweise innerhalb des Gehäuses (9) angeordnet ist,
- ein Verkleben des Bündels (4) mit den Verbindungsabschnitten (6) über ihre äußeren Zylindermantelflächen an eine Innenseite des Gehäuses (9) erfolgt.

8. Verfahren nach Anspruch 7, **gekennzeichnet durch** ein Tordieren des Bündels (4) um die Bündel-Längsachse (5) und/oder Stauchen des Bündels (4) entlang der Bündel-Längsachse (5) nach dem Verbinden der Schlauchabschnitte (3).

9. Verfahren nach Anspruch 7 oder 8, **gekennzeichnet durch** ein Einführen des Bündels (4) in das Gehäuse (9) derart, dass jeweils ein an einem Bündelende (7) angeordneter Verbindungsabschnitt (6) zumindest abschnittsweise außerhalb des Gehäuses (9) angeordnet ist.

10. Verfahren nach einem der Ansprüche 7 bis 9, **gekennzeichnet durch** ein Trennen des Bündels (4) an dem mindestens einen Verbindungsabschnitt (6), insbesondere senkrecht zur Bündel-Längsachse (5).

## Claims

1. Heat exchanger for an oxygenator, comprising a plurality of tube sections (3) each having a longitudinal tube axis (2), wherein
- the tube sections (3) are arranged in a bundle (4) having a longitudinal bundle axis (5) and spaces are formed between the tube sections (3),
- the tube sections (3) are connected to each other in at least one connecting section of the bundle (4) by joining by way of chemical and/or physical bonded joints,
- two connecting elements (6) are provided, which each are arranged at a bundle end (7),
- wherein the connecting elements (6) are designed cylindrically,
- the bundle (4) is at least partially arranged in a housing (9),
- the individual tube sections (3) are directly connected to each other by adhesive bonding, whereby the bundle (4) is formed independently and inherently dimensionally stable,
- the spaces are free of flow obstacles,
**characterized in that**
- the prefabricated, dimensionally stable bundle (4) is adhesively bonded to an inner side of the housing (9) at the connecting sections (6) via their outer cylinder surfaces.

2. Heat exchanger according to claim 1, **characterized in that** the tube sections (3) are made of plastic, in particular polyurethane.

3. Heat exchanger according to any of the preceding claims, **characterized in that** the longitudinal tube axes (2) form an angle (a) with the longitudinal bundle axis (5) which is greater than 10°, in particular greater than 20° and in particular greater than 30°.

4. Heat exchanger according to any of the preceding claims, **characterized in that** the longitudinal tube axes (2) at least sectionally are arranged non-linearly, in particular curved.

5. Heat exchanger according to any of the preceding claims, **characterized in that** the bundle (4) is designed cylindrically and has a non-variable cross-sectional surface in particular along the longitudinal bundle axis (5).

6. Heat exchanger according to any of the preceding claims, **characterized by** a perpendicular distance (D) between outer walls (10) of two neighboring tube sections (3), which is at most 200 µm, in particular at most 100 µm.

7. A method for producing a heat exchanger according to any of the preceding claims, comprising the method steps:
- providing a plurality of tube sections (3), each having a longitudinal tube axis (2);
- arranging the tube sections (3) to form a bundle (4) having a longitudinal bundle axis (5), with spaces formed between the tube sections (3), which are free of flow obstacles,
- connecting the tube sections (3) to each other on at least one connecting section (6) of the bundle (4) by joining the tube sections (3) by way of chemical and/or physical bonded joints,
- providing a second connecting section of this type,
**characterized in that**
- the individual tube sections (3) are directly connected to each other by adhesive bonding, so that the bundle (4) is formed independently and inherently dimensionally stable,
- an insertion of the prefabricated dimensionally stable bundle (4) into a housing (9) takes place in such a way that a connecting section (6) arranged at a bundle end (7) is arranged at least sectionally inside the housing (9),
- an adhesively bonding of the bundle (4) to an inner side of the housing (9) at the connecting sections (6) takes place via their outer cylinder surfaces.

8. Method according to Claim 7, **characterized by** twisting of the bundle (4) about the longitudinal bundle axis (5) and/or compressing of the bundle (4) along the longitudinal bundle axis (5) after the tube sections (3) have been connected.

9. Method according to Claim 7 or 8, **characterized by** an insertion of the bundle (4) in the housing (9) in such a way that a connecting section (6) each being arranged at a bundle end (7) is arranged at least sectionally outside the housing (9).

10. Method according to Claim 7 to 9, **characterized by** a disconnection of the bundle (4) at the at least one connecting section (6), in particular perpendicular to the longitudinal bundle axis (5).

## Revendications

1. Echangeur de chaleur destiné à un oxygénateur, comprenant plusieurs sections de tuyaux (3) présentant chacune un axe de tuyau longitudinal (2), dans lequel
- les sections de tuyaux (3) sont agencées en un faisceau (4) présentant un axe de faisceau longitudinal (5) et entre les sections de tuyaux (3) des espaces intermédiaires sont formés,
- les sections de tuyaux (3) sont reliées dans au moins une section de liaison (6) du faisceau (4) en étant jointées par le biais de compositions adhésives chimiques et/ou physiques,
- deux sections de liaison (6) sont prévues, étant chacune agencée sur une extrémité de faisceau (7),
dans lequel les sections de liaison (6) sont conçues de manière cylindrique,
- le faisceau (4) au moins en sections est agencé dans un boîtier (9),
- les sections de tuyaux (3) individuelles sont directement reliées par collage, ce qui permet au faisceau (4) d'être conçu de manière autonome et intrinsèquement indéformable,
- les espaces intermédiaires sont exempts d'obstacles à l'écoulement,
**caractérisé en ce que**
- le faisceau (4) préfabriqué et indéformable est collé sur les sections de liaison (6) par leurs surfaces extérieures d'enveloppe du cylindre sur une face intérieure du boîtier (9).

2. Echangeur de chaleur selon la revendication 1, **caractérisé en ce que** les sections de tuyaux (3) sont fabriquées en matière plastique, en particulier en polyuréthane.

3. Echangeur de chaleur selon l'une quelconque des revendications précédentes, **caractérisé en ce que** les axes de tuyau longitudinaux (2) forment un angle (a) avec l'axe de faisceau longitudinal (5), ledit angle étant plus grand que 10°, en particulier plus grand que 20° et en particulier plus grand que 30°.

4. Echangeur de chaleur selon l'une quelconque des revendications précédentes, **caractérisé en ce que** les axes de tuyau longitudinaux (2) sont agencés au moins en sections de manière non linéaire, en particulier de manière courbée.

5. Echangeur de chaleur selon l'une quelconque des revendications précédentes, **caractérisé en ce que** le faisceau (4) est conçu de manière cylindrique et présente une superficie de section transversale immuable en particulier de long de l'axe de faisceau longitudinal (5).

6. Echangeur de chaleur selon l'une quelconque des revendications précédentes, **caractérisé par** une distance (D) verticale de parois extérieures (10) de deux sections de tuyaux (3) avoisinantes, ladite distance étant d'au plus 200 µm, en particulier d'au plus 100 µm.

7. Procédé de fabrication d'un échangeur de chaleur selon l'une quelconque des revendications précédentes, comprenant les étapes de procédé
- mise à disposition de plusieurs sections de tuyaux (3) présentant chacune un axe de tuyau longitudinal (2),
- agencement des sections de tuyaux (3) sous forme d'un faisceau (4) présentant un axe de faisceau longitudinal (5), avec des espaces intermédiaires formés entre les sections de tuyaux (3), lesdits espaces intermédiaires étant exempts d'obstacles à l'écoulement,
- liaison des sections de tuyaux (3) l'une à l'autre sur au moins une section de liaison (6) du faisceau (4) en jointant les sections de tuyaux (3) par le biais de compositions adhésives chimiques et/ou physiques,
- fourniture d'une telle deuxième section de liaison,
**caractérisé en ce que**
- les sections de tuyaux individuelles (3) sont directement reliées par collage, ce qui permet au faisceau (4) d'être conçu de manière autonome et intrinsèquement indéformable,
- une insertion du faisceau (4) préfabriqué et indéformable dans un boîtier (9) a lieu de sorte que respectivement une section de liaison (6) agencée sur une extrémité de faisceau (7) au moins en sections est agencée à l'intérieur du boîtier (9),
- un collage du faisceau (4) aux sections de liaison (6) a lieu par leurs surfaces extérieures d'enveloppe du cylindre sur une surface intérieure du boîtier (9).

8. Procédé selon la revendication 7, **caractérisé par** une torsion du faisceau (4) autour de l'axe de faisceau longitudinal (5) et/ou un refoulement du faisceau (4) de long de l'axe de faisceau longitudinal (5) après la liaison des sections de tuyaux (3).

9. Procédé selon la revendication 7 ou 8, **caractérisé par** une insertion du faisceau (4) dans le boîtier (9) de sorte que respectivement une section de liaison (6) agencée sur une extrémité de faisceau (7) au moins en sections est agencée à l'extérieur du boîtier (9).

10. Procédé selon l'une quelconque des revendications 7 à 9, **caractérisé par** une séparation du faisceau (4) à l'au moins une section de liaison (6), en particulier à la verticale de l'axe de faisceau longitudinal (5).
